# EUROPÄISCHE PATENTSCHRIFT

(11) **EP 3 281 648 B1**
(45) Veröffentlichungstag und Bekanntmachung des Hinweises auf die Patenterteilung: **02.09.2020**
(21) Anmeldenummer: 17180933.8
(22) Anmeldetag: 12.07.2017
(51) Int. Cl.: A61L 27/04, A61L 27/50, A61L 27/58, A61L 31/02, A61L 31/18

(54) **RÖNTGENMARKER FÜR ABSORBIERBARE METALLISCHE SCAFFOLDS MIT HOHER RÖNTGENSICHTBARKEIT UND INTEGRIERTEM SELBSTPASSIVIERUNGSEFFEKT**
X-RAY MARKER FOR ABSORBABLE METALLIC SCAFFOLDS HAVING HIGH X-RAY VISIBILITY AND INTEGRATED SELF-PASSIVATION EFFECT
MARQUEUR AUX RAYONS X POUR ÉCHAFAUDAGE MÉTALLIQUE ABSORBANT PRÉSENTANT UNE RÉSISTANCE ÉLEVÉE AUX RAYONS X ET EFFET D'AUTOPASSIVATION INTÉGRÉ

(30) Priorität: 10.08.2016 DE 102016114800; 11.08.2016 DE 102016114894
(43) Veröffentlichungstag der Anmeldung: 14.02.2018
(73) Patentinhaber: Cortronik GmbH, 18119 Rostock-Warnemünde (DE)
(72) Erfinder: Bayer, Ullrich, 18209 Bad Doberan (DE); Lootz, Daniel, 18119 Rostock (DE); Grabow, Niels, 18055 Rostock (DE); Illner, Sabine, 18059 Rostock (DE); Eickner, Thomas, 18059 Rostock (DE); Schmitz, Klaus-Peter, 18119 Warnemünde (DE)
(74) Vertreter: Randoll, Sören

(56) Entgegenhaltungen:
- EP-A1- 3 165 238
- EP-A2- 2 399 619
- EP-A2- 2 457 601
- Mambaye N'diaye ET AL: "Water Absorption of Poly(methyl methacrylate) Measured by Vertical Interference Microscopy", Langmuir, vol. 28, no. 31, 27 July 2012 (2012-07-27) , pages 11609-11614, XP55615105, US ISSN: 0743-7463, DOI: 10.1021/la302260a

## Beschreibung

Die Erfindung betrifft ein medizinisches Implantat. Ein derartiges Implantat weist ein Gerüst auf, insbesondere ein Stentgerüst, das bevorzugt degradierbar ausgebildet ist, sich also nach einer Implantation definiert im Körper des Patienten während eines bestimmten Zeitraumes auflöst.

Derartige Implantate werden in der Regel mit Röntgenmarkem ausgestattet, so dass eine Lagebestimmung des implantierten Implantats mittels Röntgenaufnahmen möglich ist.

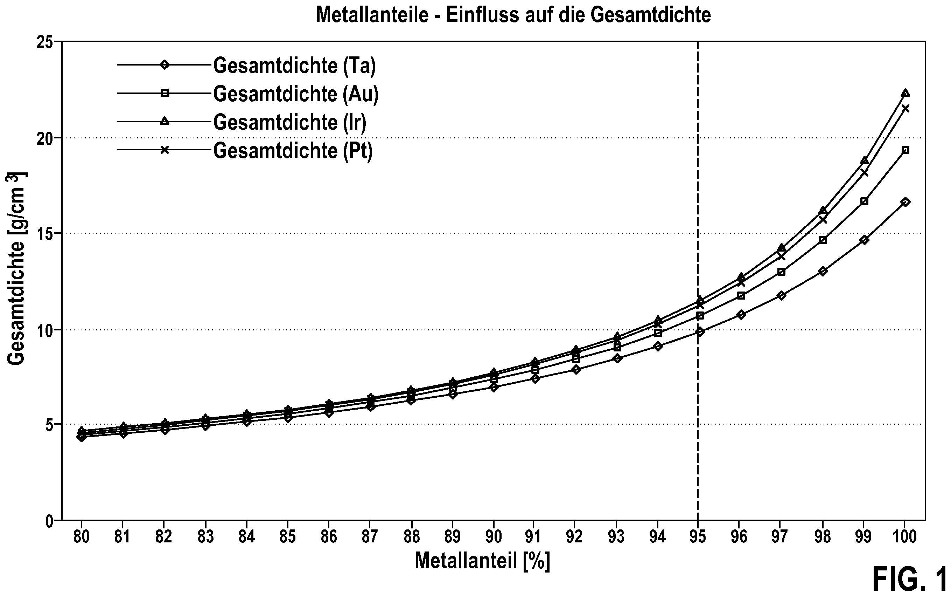

Hierzu werden z. B. gemäß EP 2 457 601 Kompositröntgenmarker verwendet, bei denen es sich um Materialkombinationen von metallischen Markerpartikeln und polymeren Klebstoffen handelt. Der Masseanteil von röntgenabsorbierenden Partikeln im Komposit bestimmt maßgeblich die Röntgensichtbarkeit. Allerdings kann deren Anteil nicht wesentlich höher als 90 Gew.% sein. Bei höheren Anteilen wird das Gemisch bereits im nicht ausgehärteten Zustand so hoch viskos, dass eine Verarbeitung wie z. B. Injektion in Aufnahmen (sogenannte Eyelets) des Gerüsts nicht mehr möglich ist. Da aufgrund der Dichteunterschiede zwischen polymeren Klebstoffen (Rho < 2 g/cm³) und röntgenabsorbierenden Metallen wie Ta (Rho > 16 g/cm³) sich die Volumenverhältnisse genau konträr zu den jeweiligen Masseverhältnissen darstellen, gestaltet sich eine weitere Optimierung dieser Technologie schwierig. Selbst wenn es gelingen sollte, 95 Gew.% an röntgenabsorbierenden Partikeln in ein polymerhaltiges Komposit einzubringen und dieses auch definiert in ein Eyelet einzubringen, könnte trotzdem - aufgrund der sich dann einstellenden Dichte des Komposites - nur die Hälfte des jeweiligen Röntgenabsorptionspotentials genutzt werden, wie in der Figur 1 dargestellt ist. Dies führt besonders im Hinblick der Realisierung geringerer Wandstärken zu einer verminderten, nicht mehr relevanten Röntgensichtbarkeit.

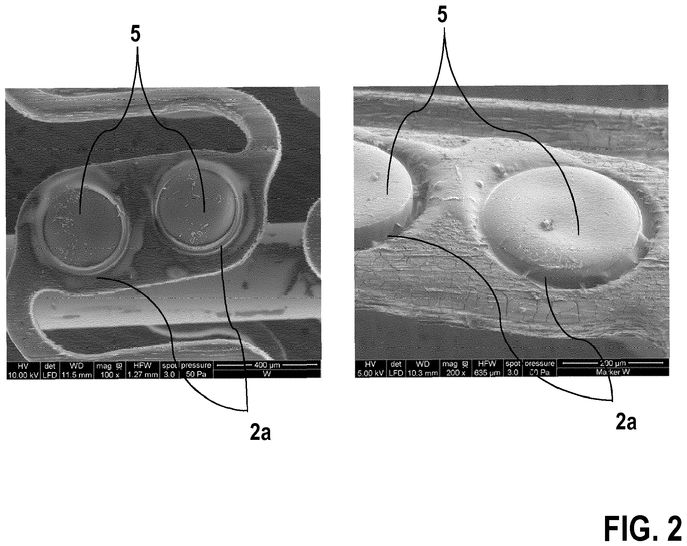

Weiterhin ist aus dem Stand der Technik die einfache Verklebung von Festkörpermarkern in die Eyelets von absorbierbaren Gerüsten (auch Scaffolds genannt) bekannt. Eine derartige Verklebung verhindert jedoch nur dann die Bildung von korrosionsbeschleunigenden Lokalelementen, wenn der Klebstoff eine permanente örtliche Trennung zwischen Marker und Scaffold während des gesamten Degradationsverlaufs sicherstellt. Dies ist regelmäßig nicht der Fall, wie z. B. in der Figur 2 dargestellt ist. Danach kann der Klebespalt 2a (ca. 20 Mikrometer) eines verwendeten Silikonklebstoffs technologisch bedingt nicht gleichmäßig über den Umfang verteilt werden. Nach der Verbringung in den Patienten, einer Dilatation und einer Verweilzeit des Gerüsts in der Gefäßumgebung von einigen Tagen bis Wochen bewirken Degradationseffekte eine Verschiebung des Röntgenmarkers 5 im jeweiligen Eyelet. Dies führt zumindest zum punktförmigen oder auch flächenhaften metallischen Direktkontakt zwischen Marker 5 und dem umgebenden Bulkmaterial des Gerüsts. Eine lokal erhöhte, rasch fortschreitende Korrosion der Markerumgebung ist die Folge. Dieser Effekt ist unbedingt zu vermeiden.

Weiterhin wird zur Verminderung des Korrosionsrisikos im Stand der Technik (z. B. EP 2 399 619) vorgeschlagen, einen Festkörperröntgenmarker mit Fremdmetallen zu ummanteln. Bei diesen Varianten wird zwar die Gefahr der Lokalelementbildung bzw. Korrosion wesentlich reduziert, allerdings ist auch hier der technologische Herstellungsaufwand sehr hoch.

Weiterhin sind aus der EP 1 973 502 mittels Form- und Kraftschluss in die Eyelets von polymeren Scaffolds eingebrachte Röntgenmarker bekannt, wobei nachträglich Lücken zwischen Marker und Scaffold mittels eines polymeren Materials verfüllt werden.

Hiervon ausgehend liegt der vorliegenden Erfindung die Aufgabe zugrunde, ein medizinisches Implantat sowie ein entsprechendes Verfahren zur Herstellung des Implantats bereitzustellen, das die Korrosionsgefahr aufgrund einer Zusammenwirkung des Gerüsts mit dem Röntgenmarker (insbesondere in einer wässrigen Umgebung) deutlich vermindert.

Diese Aufgabe wird durch ein Implantat mit den Merkmalen des Anspruchs 1 sowie durch ein Verfahren mit den Merkmalen des Anspruchs 9 gelöst. Vorteilhafte Ausführungsformen dieser Aspekte der Erfindung sind in den entsprechenden Unteransprüchen angegeben und werden nachfolgend beschrieben.

Gemäß Anspruch 1 wird ein medizinisches Implantat vorgeschlagen, mit:
- einem Gerüst, das zumindest eine Aufnahme für einen Röntgenmarker aufweist, wobei das Gerüst ein erstes Metall aufweist,
- zumindest einem in der Aufnahme angeordneten monolithischen Röntgenmarker, wobei der Röntgenmarker ein zweites Metall aufweist,
- wobei erfindungsgemäß vorgesehen ist, dass der Röntgenmarker ein zweites Metall aufweist, und wobei auf den Röntgenmarker zum Verhindern eines korrosionsfördernden Kontakts zwischen dem Röntgenmarker und dem Gerüst eine elektrisch isolierende Beschichtung aufgebracht ist, dadurch gekennzeichnet dass die Beschichtung derart quellfähig ausgebildet ist, dass sie bei einem Kontakt mit einem Wasser enthaltenden Medium eine Volumenzunahme erfährt.

Der Röntgenmarker ist monolithisch aufgebaut, d. h., er weist einen zusammenhängenden Grundkörper auf und besteht in diesem Fall nicht aus einer Vielzahl an Markerpartikeln, die durch einen separaten Klebstoff zusammengehalten werden.

Bei dem Gerüst handelt es sich bevorzugt um ein Stentgerüst, das z. B. dazu eingerichtet und vorgesehen ist, in ein Blutgefäß eines Patienten implantiert zu werden. Das Stentgerüst weist dabei eine Vielzahl miteinander verbundener Streben auf, die die Zellen des Stentgerüstes bilden. Das Stentgerüst weist dabei zwei Enden auf, die eine Einlass- bzw. Auslassöffnung des Stentgerüstes umgeben, durch die Blut in einen vom Stentgerüst umgebenen Innenraum des Stentgerüstes einströmen bzw. wieder ausströmen kann. Die besagte Aufnahme ist bevorzugt als Durchgangsöffnung einer Strebe des Gerüsts bzw. Stentgerüstes ausgebildet, die bevorzugt an einem der Enden angeordnet ist. Bevorzugt sind an einer solchen Strebe zwei benachbarte Aufnahmen vorgesehen, die jeweils als Durchgangsöffnung ausgebildet sind und in die jeweils ein erfindungsgemäßer Röntgenmarker eingebracht wird. Bevorzugt sind zwei derartig benachbarte Aufnahmen, die auch als Eyelets bezeichnet werden, an beiden Enden des Stentgerüstes vorgesehen. Auf diese Weise kann mittels der Röntgenmarker, die dazu konfiguriert sind, in einer Röntgenaufnahme einen deutlichen Kontrast zu erzeugen, die genaue Raumlage und Position des Stents ermittelt werden. Ein Eyelet kann dabei erfindungsgemäß jede für die entsprechende Anwendung benötigte Form annehmen, beispielsweise rund, oval, trapezförmig oder quadratisch. Insbesondere besitzt das Eyelet eine Größe von 10 bis 1000 µm, von 10 bis 100 µm und von 20 bis 200 µm.

Gemäß der Erfindung ist vorgesehen, dass die Beschichtung derart quellfähig ausgebildet ist, dass sie bei Kontakt mit einem Wasser enthaltenden Medium eine Volumenzunahme erfährt und dabei den Röntgenmarker zur Vermeidung des besagten Kontakts vom Gerüst beabstandet.

Weiterhin ist gemäß einer Ausführungsform des erfindungsgemäßen Implantats vorgesehen, dass der Röntgenmarker eine Oberfläche mit einer Porenstruktur aufweist, wobei der Porendurchmesser bevorzugt im Bereich von 0,5 Mikrometer bis 3 Mikrometer liegt, oder dass der Kern eine Oberflächenstruktur aufweist, die bevorzugt einen Oberflächenrauheitswert Ra im Bereich von 0,5 Mikrometer bis 3 Mikrometer aufweist.

Weiterhin ist gemäß einer Ausführungsform des erfindungsgemäßen Implantats vorgesehen, dass die Oberfläche zur Herstellung der Porenstruktur plasmachemisch oxidiert wird oder per Beaufschlagung mit Mikrostrahlen mechanisch bearbeitet wird.

Weiterhin ist gemäß einer Ausführungsform des erfindungsgemäßen Implantats vorgesehen, dass die Beschichtung in den Poren oder in der Oberflächenstruktur aufgenommen ist und dazu ausgebildet ist, bei einem Kontakt mit einem Wasser enthaltenden Medium aus den Poren oder der Oberflächenstruktur herauszutreten, so dass bevorzugt eine Zentrierung des Röntgenmarkers in der Aufnahme sowie insbesondere eine Selbstpassivierung des Röntgenmarkers erfolgt.

Weiterhin ist gemäß einer Ausführungsform des erfindungsgemäßen Implantats vorgesehen, dass der Röntgenmarker in der Aufnahme festgeklebt ist. Hierzu kann ein separater Klebstoff verwendet werden oder die besagte Beschichtung bildet selbst den notwendigen Klebstoff.

Weiterhin ist gemäß einer Ausführungsform des erfindungsgemäßen Implantats vorgesehen, dass das zweite Metall eines der folgenden Metalle ist: Wolfram, Tantal, Gold, Platin, Iridium.

Weiterhin ist gemäß einer Ausführungsform des erfindungsgemäßen Implantats vorgesehen, dass das Gerüst ein degradierbares Gerüst ist, das dazu ausgebildet ist, sich nach einer Implantation in einen menschlichen oder tierischen Körper innerhalb einer vordefinierbaren Zeitspanne vollständig aufzulösen.

Weiterhin ist gemäß einer Ausführungsform des erfindungsgemäßen Implantats vorgesehen, dass das erste Metall Magnesium ist, wobei das Magnesium Bestandteil einer Magnesiumlegierung ist, die das Gerüst aufweist oder aus der das Gerüst besteht.

Weiterhin ist gemäß einer Ausführungsform des erfindungsgemäßen Implantats vorgesehen, dass die Beschichtung einen oder mehrere der folgenden Stoffe aufweist: ein Polymer, insbesondere ein möglicherweise quellfähiges, unlösliches, vernetztes Polymer wie u. a. Polyacrylate, Polyacrylsäure (PAA), Polylaurinsäurevinylester, Polyvinylimidazol, Polyvinylpyrrolidon, Polyglycolsäure (PGA), Polyethylenglykol (PEG), Hyaluronsäure (HA), Polyharnstoffe, Polyurethane, Wasserglas, Cyanacrylat, Paraffin in Kombination mit einem geeigneten Vernetzer und ggf. einem geeigneten Lösungsmittel.

Weiterhin wird die erfindungsgemäße Aufgabe durch ein Verfahren zur Herstellung eines medizinischen Implantats mit den Merkmalen des Anspruchs 9 gelöst, wobei ein Gerüst bereitgestellt wird, das zumindest eine Aufnahme für einen monolithischen Röntgenmarker aufweist, wobei das Gerüst ein erstes Metall aufweist, und wobei ein, ein zweites Metall aufweisender monolithischer Röntgenmarker bereitgestellt wird, auf den eine elektrisch isolierende Beschichtung mittels Kontaktierung mit einer Beschichtungslösung aufgebracht wird, wobei die Beschichtungslösung ein quellfähiges Polymer aufweist und wobei der beschichtete Röntgenmarker in der Aufnahme festgeklebt wird.

Das Gerüst bzw. der Röntgenmarker können die weiter oben bzw. die in den Ausführungsbeispielen beschriebenen Merkmale aufweisen.

Gemäß einer Ausführungsform des erfindungsgemäßen Verfahrens ist vorgesehen, dass der Röntgenmarker durch eine Behandlung der Oberfläche des Röntgenmarkers eine Poren- oder Oberflächenstruktur erhält.

Gemäß der Erfindung ist vorgesehen, dass der Röntgenmarker zum Aufbringen der Beschichtung mit einer Beschichtungslösung kontaktiert wird.

Weiterhin ist gemäß einer Ausführungsform des erfindungsgemäßen Implantats vorgesehen, dass diese Beschichtungslösung ein Hydrogelmonomer, einen Vernetzer, einen Polymerisationsinitiator und einen Polymerisationskatalysator aufweist.

Gemäß einer Ausführungsform der Erfindung können als Hydrogelmonomer Monomere wie beispielsweise Acrylate, Laurinsäurevinylester, Vinylimidazol, Vinylpyrrolidon, Diisocyanate u. a. zum Einsatz kommen, bevorzugt vinyloge Monomere. Weiterhin können gemäß einer Ausführungsform der Erfindung Vernetzer in Form von N,N'-Methylen-bis(acrylamid) (MBAA), Glutardialdehyd, 1-Ethyl-3-(3-dimethylaminopropyl)-carbodiimid/ N-Hydroxysuccinimid (EDC/NHS) oder andere verwendet werden. Weiterhin kann gemäß einer Ausführungsform Tetramethylethylendiamin (TEMED) als Polymerisationskatalysator verwendet werden.

Weiterhin können gemäß einer Ausführungsform als Polymerisationsinitiator Ammoniumpersulfat (APS) oder Azobis(isobutyronitril) (AIBN) verwendet werden

Gemäß einer Ausführungsform ist weiterhin vorgesehen, dass die Polymerisation auf der Oberfläche des Röntgenmarkers thermisch oder photochemisch aktiviert werden kann.

Weiterhin kann die Polymerisation ergänzend gemäß einer Ausführungsform katalytisch unterstützt werden, z. B. durch eine entsprechende Zusammensetzung der Oberfläche des Röntgenmarkers, die z. B. durch eine amorphe Oxidschicht gebildet sein kann (z. B. WO₃ oder Natriumwolframat als Schichtbestandteil).

Gemäß der Erfindung ist vorgesehen, dass der beschichtete Röntgenmarker in die zugeordnete Aufnahme des Gerüsts geklebt wird. Hierbei kann als Klebstoff z. B. ein Silikon aufweisender Klebstoff verwendet werden.

Gemäß der Erfindung ist vorgesehen, dass die Beschichtungslösung ein quellfähiges Polymer aufweist, wobei die Beschichtungslösung weiterhin insbesondere einen Klebstoff aufweist.

Gemäß einer Ausführungsform kann die Beschichtungslösung ein Polymer bzw. einen gelösten Superabsorber wie z. B. Polyacrylsäure (PAA), Polyglycolsäure (PGA), Polyethylenglykol (PEG), Hyaluronsäure (HA) oder andere derartige Stoffe aufweisen. Weiterhin kann die Beschichtungslösung neben einem oder mehreren dieser oder anderer geeigneter Polymere ELA-NCO oder Wasserglas oder ein Cyanacrylat, oder verdünnte wässrige oder lösungsmittelhaltige Gemische (z. B. eine chloroformhaltige Paraffinlösung) aufweisen. Paraffin oder Wasserglas sind mit Vorteil elektrisch isolierend und inert.

Gemäß einer Ausführungsform der Erfindung wird die Beschichtungslösung mit dem quellfähigen Polymer gleichzeitig als Klebstoff verwendet, d.h. der Röntgenmarker wird gemäß einer Ausführungsform mittels der Beschichtungslösung auch direkt ohne Zusatz eines (Silikon)klebstoffes mit der Aufnahme verklebt.

Gemäß der Erfindung ist vorgesehen, dass der Röntgenmarker in der Aufnahme festgeklebt wird, wobei vorzugsweise ein hierzu verwendeter Klebstoff durch die Beschichtung bzw. die Beschichtungslösung gebildet wird.

Generell sind das entstehende quellfähige Polymergel (Hydrogel) oder der quellfähige Kleberersatz vorzugsweise biokompatibel und quellen bei Anwesenheit von wässrigen (auch Chlorid haltigen) Lösungen beziehungsweise Blut auf.

Zusammenfassend ermöglicht die vorliegende Erfindung durch die quellfähige, elektrisch isolierende Beschichtung die Verwendung von Solidröntgenmarkern (monolithische Röntgenmarker), deren bisherige Anwendung bei degradierbaren Scaffold- bzw. Gerüstmaterialien, wie Magnesiumlegierungen, erhöhte Korrosionsraten und daraus resultierend eine erheblich verkürzte Standzeit zur Folge hatte. Die Gefahr der unbeabsichtigt und plötzlich auftretenden Bildung von Lokalelementen während der klinisch relevanten Stützzeit und die daraus resultierende nicht kalkulierbare Korrosion mit verstärkter lokaler Wasserstoffbildung und damit verbundener möglicher unerwünschter biologischer Reaktionen tritt nicht mehr auf bzw. wird deutlich unterdrückt.

Der erfindungsgemäße Quellungseffekt bewirkt eine wesentliche Volumenvergrößerung der Beschichtung in dessen Folge das Polymer/Hydrogel aus der porösen bzw. strukturierten Oberfläche des Röntgenmarkers heraustritt.

Beim Auftreffen des Polymers auf die Wandung der mindestens einen Aufnahme bzw. Eyelets wird einerseits der Quelleffekt in andere Richtungen umgelenkt, andererseits findet im Klebespalt (z. B. ca. 20 µm) eine Selbstzentrierung des Röntgenmarkers in der Aufnahme statt und verhindert den Metall-Metall-Kontakt zwischen dem ersten und dem zweiten Metall. Die korrosive Umgebung selbst bewirkt damit insbesondere eine Selbstpassivierung. Der Degradationsverlauf bis zur völligen Umwandlung des Gerüsts in seine Korrosionsprodukte wird nicht unterbrochen.

Die Erfindung ermöglicht letztlich eine Vereinfachung des Markermontageprozesses, da eine Erhöhung der Fertigungstoleranzen möglich ist. Durch die nachträgliche Quellung kommt es insbesondere zur Selbstzentrierung des Röntgenmarkers, wodurch auch unvollständig zentrierte Röntgenmarker akzeptiert werden können.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine Darstellung der sich ergebenden Kompositdichte als Funktion des Metallanteils. Die senkrechte Linie stellt den maximalen Metallanteil dar, der als Komposit derzeit technologisch herstellbar ist;
- Fig. 2: einen eingeklebter Solidmarker mit ungleichmäßig gefülltem Klebespalt;
- Fig. 3: eine poröse plasmachemisch oxidierte Oberfläche eines Röntgenmarkers aus (links) Tantal und (rechts) Wolfram;
- Fig. 4: eine Ansicht eines erfindungsgemäßen Implantats mit benachbarten Aufnahmen (Eyelets) für Röntgenmarker; und
- Fig. 5: eine schematische Ansicht eines erfindungsgemäßen Eyelets.

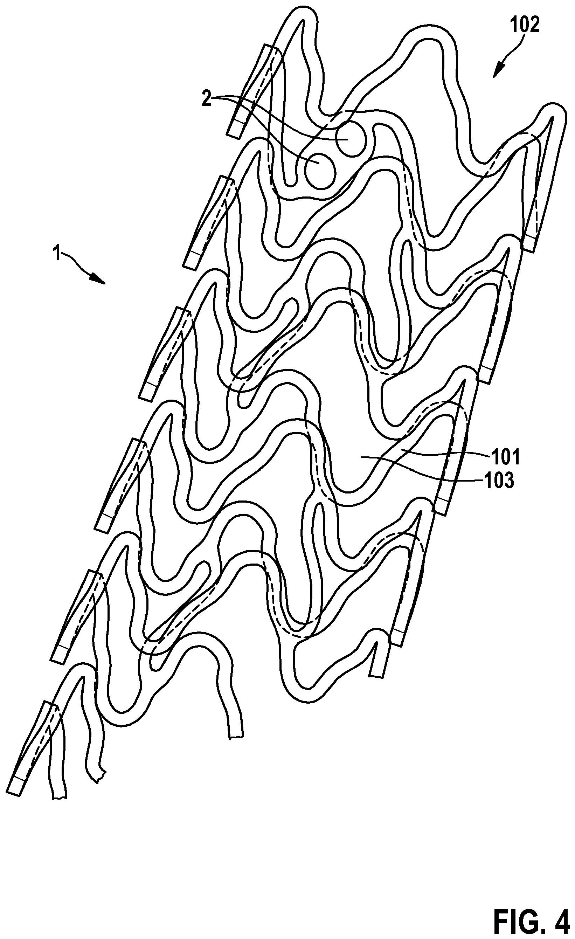

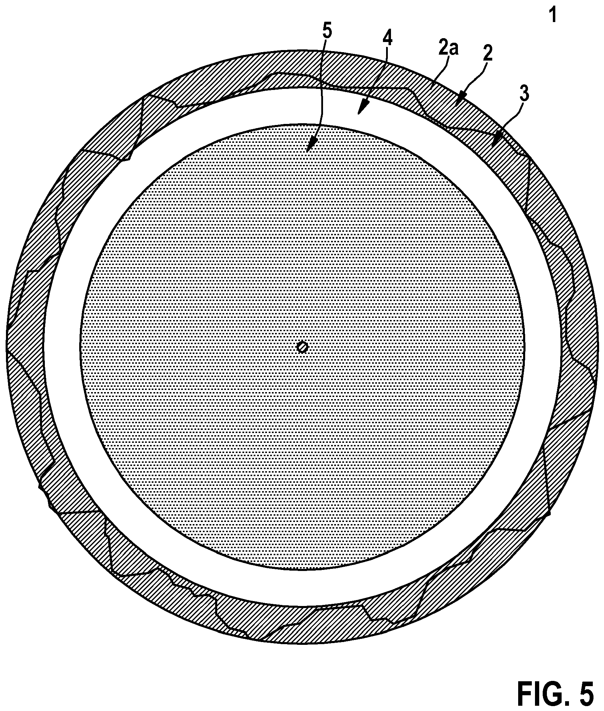

Die Figur 4 zeigt im Zusammenhang mit der Figur 5 ein erfindungsgemäßes medizinisches Implantat, mit einem Gerüst 1, das zumindest eine Aufnahme 2 für einen Röntgenmarker 5 aufweist,

Bei dem Gerüst 1 handelt es sich insbesondere gemäß Figur 4 um ein Stentgerüst, das z. B. dazu eingerichtet und vorgesehen ist, in ein Blutgefäß eines Patienten implantiert zu werden. Das Stentgerüst 1 weist dabei eine Vielzahl miteinander verbundener Streben 101 auf, die Zellen des Stentgerüstes 1 bilden. Das Stentgerüst weist dabei zwei Enden 102 auf (in der Figur 4 ist nur ein Ende gezeigt), die eine Einlass- bzw. Auslassöffnung des Stentgerüstes 1 umgeben, durch die Blut in einen vom Stentgerüst 1 umgebenen Innenraum 103 des Stentgerüstes 1 einströmen bzw. wieder ausströmen kann. Die besagte mindestens eine Aufnahme 2 ist bevorzugt als Durchgangsöffnung einer Strebe 101 des Gerüsts bzw. Stentgerüstes 1 ausgebildet. Gemäß Figur 4 sind z. B. zwei benachbarte Aufnahmen 2 bzw. Eyelets 2 an einem der Enden 102 oder an beiden Enden 102 angeordnet.

Sind die Röntgenmarker 5 in der jeweiligen Aufnahme 2 angeordnet, wird ein umlaufender Klebespalt 2a definiert, der schematisch in der Figur 5 gezeigt ist.

Das Gerüst 1 weist ein erstes Metall auf, vorzugsweise Magnesium, das in einer das Gerüst 1 bildenden Magnesiumlegierung vorliegt, die z. B. eine Zusammensetzung aufweisen kann, wie sie in DE 101 18 603 A1 beschrieben ist, und zumindest einen in der Aufnahme 2 angeordneten Röntgenmarker 5. Der mindestens eine Röntgenmarker 5 bzw. die mehreren Marker 5, der bzw. die in der jeweiligen Aufnahme 2 angeordnet ist bzw. sind, weisen weiterhin ein zweites Metall auf, das eines der hierin beschriebenen Metalle sein kann (siehe oben sowie Ausführungsbeispiele weiter unten).

Unter Magnesiumlegierung wird hier ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%, ganz besonders bevorzugt mehr als 90 Gew.%. Vorzugsweise enthält die biokorrodierbare Magnesiumlegierung des Kerns Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Dem Fachmann ist eine Vielzahl von Magnesiumlegierungen bekannt. Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Insbesondere bevorzugt ist eine Magnesiumlegierung der Zusammensetzung Yttrium 3,7 - 5,5 Gew.%, Neodym 1,5 - 3 Gew.%, Zirkonium 0,3 - 1 Gew.%, Gadolinium 0,4 - 0,55 Gew.%, Dysprosium 0,4 - 0,55 Gew.%, Eisen <0,006 und Rest < 1 Gew.%, wobei Magnesium den auf 100 Gew.% fehlenden Anteil an der Legierung einnimmt. Weiterhin bevorzugt sind Legierungen auf Basis von Magnesium der Form Mg-Zn-Al, Mg-Zn-Ca, Mg-Zn oder Mg-Al, wobei diese Legierungen höchstens Spuren von Seltenerdmetalle oder weiteren Elementen aufweisen. Bevorzugt ist eine Magnesiumlegierung der Zusammensetzung Zink 1,5 -7,0 Gew.%, Aluminium 0,5 - 10,0 Gew.%, Rest < 0,0063 Gew.%, wobei Magnesium den auf 100 Gew.% fehlenden Anteil an der Legierung einnimmt. Weiterhin bevorzugt ist eine Magnesiumlegierung der Zusammensetzung Zink 3,0 - 7,0 Gew.%, Kalzium 0,001 - 0,6 Gew.%, Rest < 0,0048 Gew.%, wobei Magnesium den auf 100 Gew.% fehlenden Anteil an der Legierung einnimmt. Weiterhin bevorzugt ist eine Magnesiumlegierung der Zusammensetzung Aluminium 1,0 - 10,0 Gew.%, Rest < 0,0048 Gew.%, wobei Magnesium den auf 100 Gew.% fehlenden Anteil an der Legierung einnimmt.

Erfindungsgemäß ist nun vorgesehen, dass der mindestens eine Röntgenmarker 5 zum Verhindern des eingangs beschriebenen korrosionsfördernden Kontakts zwischen dem zweiten Metall des mindestens einen Röntgenmarkers 5 und dem ersten Metall (bzw. weiteren Metallen) des Gerüsts 1 eine elektrisch isolierende Beschichtung 3 aufweist, die quellfähig ausgebildet ist, so dass sie bei einem Kontakt mit einem Wasser enthaltenden Medium (insbesondere bei Blutkontakt nach einer Implantation) eine Volumenzunahme erfährt.

Diese Quellung bei Kontakt mit dem besagten Medium bewirkt einerseits eine Zentrierung des Röntgenmarkers in der Aufnahme sowie eine entsprechende Beabstandung von einem Rand der Aufnahme, so dass ein gleichmäßiger Klebespalt 2a resultiert. Des Weiteren bewirkt die Volumenzunahme gewissermaßen eine Selbstpassivierung des Markers 5 bei Kontakt mit dem besagten wässrigen Medium. Da dieser durch die Volumenzunahme der Beschichtung wirksam (Korrosion vermeidend) abgeschirmt wird.

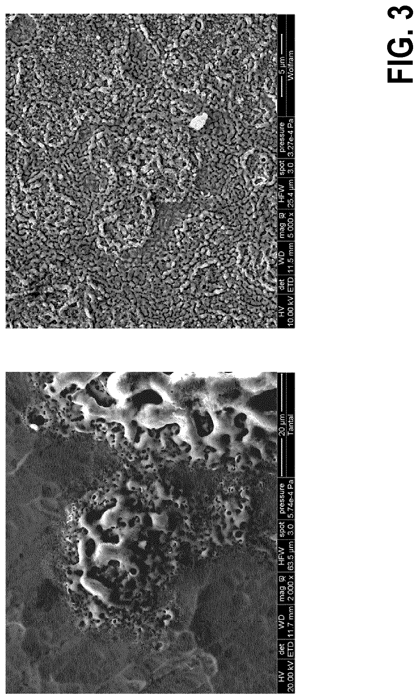

Zur besseren Anhaftung der Beschichtung 3 ist bevorzugt vorgesehen, dass die Oberfläche 4 des mindestens einen Röntgenmarkes 5 eine Oberflächenaufrauhung erhält, indem der Solidmarker 5 aus W (oder Ta) gefertigt wird und plasmachemisch oxidiert wird (siehe Figur 3) oder als Edelmetallmarker 5 (aus Au oder Pt) ausgebildet wird, der per Mikrostrahlen mechanisch behandelt wird. Die bereits vorhandene, natürliche kristalline Oxidschicht (wenige nm dick) bei W und Ta wird hierbei durch eine amorphe Oxidschicht (mehrere µm dick) ersetzt. Wolfram- und Tantal-Marker 5 besitzen danach eine Porenstruktur mit Porendurchmessern zwischen 0,5 und 3 µm, die Porentiefe ist insbesondere etwas kleiner als die Schichtdicke. Die Edelmetallmarker 5 weisen weiterhin Oberflächenrauheitswerte zwischen Ra 0,5 und 3 µm auf.

Die so behandelten Marker 5 mit einem Durchmesser von z. B. ca. 320 µm werden gemäß einem ersten Beispiel der Erfindung in eine Lösung zur quellfähigen Beschichtung getaucht.

Durch Anlegen eines Unterdruck, beispielsweise mit Hilfe eines Exsikkators, von unter 200 mbar werden die Poren mit dem quellfähigen Polymer oder der dieses durch Polymerisation bildenden Monomerlösung gefüllt und die Luftblasen entfernt. Danach erfolgt eine Trocknung, Polymerisation (Aushärten an Luft oder thermisch aktiviert) oder sofortige Verklebung in das jeweilige Eyelet 2.

Gemäß einem Beispiel der Erfindung besteht die Lösung zur quellfähigen Beschichtung aus:
einer Additivlösung aus einem Hydrogelmonomer, Vernetzer, Initiator und Polymerisationskatalysator. Als Hydrogelmonomer werden bevorzugt vinyloge Monomere wie beispielsweise Laurinsäurevinylester, Vinylimidazol, Vinyl¬pyrrolidon, u.a. eingesetzt. Als Vernetzer können z. B. N,N'-Methylen¬bis(acrylamid) (MBAA), Glutardialdehyd, 1-Ethyl-3-(3-dimethyl¬aminopropyl)¬carbodiimid/ N-Hydroxy¬succinimid (EDC/NHS) oder andere fungieren. Als Initiatoren können z. B. Ammoniumpersulfat (APS) oder Azo-bis(isobutyronitril) (AIBN) genutzt werden. Als Polymerisationskatalysator kann z. B. Tetramethylethylendiamin (TEMED) verwendet werden. Hierbei kann die Polymerisation auf der Markeroberfläche 4 thermisch oder photochemisch aktiviert werden oder katalytisch, durch die Zusammensetzung der amorphen Oxidschicht (z. B. WO₃ oder Natriumwolframat als Schichtbestandteil) unterstützt werden. Eine nachträgliche Verklebung des so isolierten Markers 5 im Eyelet 2 erfolgt z. B. mit einem geeigneten Klebstoff.

Gemäß einem alternativen Beispiel kann die Beschichtungslösung aus einem quellfähigen Polymer bestehen, das auch als Kleber eingesetzt werden kann und diesen ggf. ersetzt. Innerhalb dieses Haftgels wird ein quellfähiges Polymer mit einem Kleberersatzstoff kombiniert. Das Haftgel kann aus einem gelösten Superabsorber wie beispielsweise Polyacrylsäure (PAA), Polyglycolsäure (PGA), Polyethylenglykol (PEG), Hyaluronsäure (HA) oder anderen geeigneten Polymeren, und aus Polyharnstoffen oder Polyurethanen oder Wasserglas oder Cyanacrylaten oder verdünnten wässrigen oder lösungsmittelhaltigen Gemischen (z. B. chloroformhaltige Paraffinlösung) bestehen.

Diese Lösung zur quellfähigen Beschichtung dient bevorzugt gleichzeitig als Kleber, wodurch der jeweilige Marker 5 ggf. auch direkt ohne Zusatz eines Silikonklebstoffes im zugeordneten Eyelet 2 verklebt werden kann.

Generell sind das entstehende quellfähige Polymergel (Hydrogel) oder der quellfähige Kleberersatz biokompatibel und quellen bei Anwesenheit von wässrigen (auch Chlorid haltigen) Lösungen beziehungsweise Blut auf.

Gemäß einem weiteren Beispiel der Erfindung wurde ein Gerüst 1 mit einem Röntgenmarker 5 aus Wolfram bereitgestellt (vgl. Figuren 4 und 5), der eine mittels plasmachemischer Oxidation erzeugte makroporöse Oberfläche 4 aus Wolframoxid aufweist.

Das Gerüst 1 weist dabei eine degradierbare Magnesiumlegierung (vgl. Fig. 4) auf, die jeweils am distalen und am proximalen Ende 102 ein Doppeleyelet 2 aufweist (d. h. zwei benachbarte Aufnahmen in Form von Durchgangsöffnungen 2 einer Strebe 101), das für eine Befüllung mit Röntgenmarkern 5 vorgesehen ist. Der Durchmesser eines Eyelets 2 beträgt z. B. ca. 350 µm.

In diese Eyelets 2 werden runde Massivmarker 5 aus Wolfram montiert. Die Dicke dieser Wolframmarker ist identisch mit der Wanddicke des Scaffolds 1 und beträgt beispielsweise 100 µm. Der Durchmesser ist ca. 20 µm geringer als der Eyeletdurchmesser. Der Wolframmarker 5 wurde vorher in einem Schwefel- und Phosphorsäure enthaltenden Elektrolyt plasmachemisch oxidiert. Die Oxidation des chemisch stabilen Elements Wolfram wird durch die bei Badspannungen > 180 V entstehenden lokal begrenzten Plasmaentladungen hervorgerufen. Dabei rastern einzelne Plasmaentladungen die Wolframoberfläche systematisch ab. Die Oberfläche des Markers 5 erhält dadurch eine verfahrenstypische poröse Oberfläche, die zum größten Teil aus WO₃ besteht. Die Oxidschichtdicke beträgt zwischen 3 und 6 µm. Aufgrund des Konversionscharakters der Plasmaentladungen bleibt die ursprüngliche Außengeometrie des Markers 5 erhalten und die Oxidschicht weist aufgrund der stofflichen Verzahnung mit dem darunter liegenden metallischen Substrat eine hohe Haftfestigkeit auf (vgl. Fig. 5). Diese Oberflächenstrukturierung führt auch zu einer wesentlichen Vergrößerung der realen Oberfläche mindestens um den Faktor 2.

Dies ist eine wesentliche Voraussetzung für eine hohe Benetzungsfähigkeit. Der so behandelte Marker 5 wird anschließend in eine Lösung zur quellfähigen Beschichtung getaucht, die z. B. gemäß einem der oben genannten Beispiele ausgeführt sein kann. Um die in der porösen Oberfläche 4 enthaltenden Luftblasen zu entfernen, erfolgt dieser Tauchprozess in einem Exsikkator, der mit Unterdruck < 200 mbar beaufschlagt wird. Nach einer Behandlungszeit zwischen 2 und 5 min ist der Entgasungsprozess abgeschlossen und es sind keine aufsteigenden Luftblasen mehr sichtbar. Die Markeroberfläche 4 ist mit dem quellfähigen Polymer 3 benetzt und wird aus der Beschichtungslösung entnommen. Gegebenenfalls erfolgt anschließend eine thermisch initiierte Polymerisation bei > 50°C, eine Lösungsmittelentfernung im Vakuumtrockenschrank oder eine direkte Verklebung im jeweiligen Eyelet 2.
Nach diesem Prozess wird der Marker 5 in das Eyelet 2 positioniert. Gegebenenfalls wurde die Eyeletumgebung vorher mit der zuvor (zum Beschichten des jeweiligen Markers 5) verwendeten Lösung zur quellfähigen Beschichtung belegt (siehe oben). Nach der Positionierung des jeweiligen Markers 5 im zugeordneten Eyelet 2 erfolgt die finale thermische Behandlung, die zu einer Polymerisation der Beschichtung auf der Röntgenmarkeroberfläche führt und eine ausreichende Haltekraft des Markers 5 im Eyelet 2 bewirkt. Danach erfolgt insbesondere die Montage des Scaffolds 1 in ein Kathetersystem.

Während der vaskulären Intervention des Scaffolds 1 kommt die Umgebung des Röntgenmarkers 5 in Kontakt mit Blut. Daraufhin setzt ein Quellprozess des polymeren Hydrogels 3 ein. Dieser führt zu einer Volumenvergrößerung und einem Heraustreten des Hydrogels 3 aus der Markeroberfläche 4. Dies trifft auch auf die Schnittkanten des Markers 5 zu. Dieser Selbstversiegelungseffekt stellt eine Verkapselung des Markers 5 und eine Isolation dessen zum übrigen Scaffold 1 dar. Zudem werden technologisch bedingte Unzulänglichkeiten beim Prozess der Markerpositionierung durch eine nachträgliche Selbstzentrierung des Markers 5 im Eyelet 2 ausgeglichen. Somit wird sichergestellt, dass kein Metall-/Metallkontakt entsteht, die Lokalelementbildung sicher unterbunden wird und keine Beeinflussung des weiteren Degradationsverlaufs des Scaffolds 1 stattfindet.

Gemäß einem weiteren Beispiel der Erfindung wurde ein Scaffold 1 mit einem Röntgenmarker 5 aus Tantal bereitgestellt, der eine mittels plasmachemischer Oxidation erzeugte makroporöse Oberfläche 4 aus Tantaloxiden aufweist. Das verwendete Gerüst (Scaffold) 1 aus einer degradierbaren Magnesiumlegierung (vgl. Figur 4) hat wiederum jeweils am distalen und am proximalen Ende 102 ein Doppeleyelet 2, das für eine Befüllung mit Röntgenmarkern 5 vorgesehen ist. Der Durchmesser eines Eyelets 2 beträgt z. B. ca. 350 µm. In diese Eyelets 2 werden runde Massivmarker 5 aus Tantal montiert. Die Dicke dieser Tantalmarker 5 ist identisch mit der Wanddicke des Scaffolds 1 und beträgt beispielsweise 100 µm. Der Durchmesser ist ca. 20 µm geringer als der Eyeletdurchmesser. Der jeweilige Tantalmarker 5 wurde vorher in einem Schwefel- und Phosphorsäure enthaltenden Elektrolyt plasmachemisch oxidiert. Die Oxidation des chemisch stabilen Elements Tantal wird durch die bei Badspannungen > 180 V entstehenden lokal begrenzten Plasmaentladungen hervorgerufen. Dabei rastern einzelne Plasmaentladungen die Tantaloberfläche systematisch ab. Die Oberfläche des Markers 5 erhält dadurch eine verfahrenstypische poröse Oberfläche 4, die zum größten Teil aus Tantalpentoxid Ta₂O₅ besteht. Die Oxidschichtdicke beträgt zwischen 3 und 6 µm. Aufgrund des Konversionscharakters der Plasmaentladungen bleibt die ursprüngliche Außengeometrie des Markers 5 erhalten und die Oxidschicht weist aufgrund der stofflichen Verzahnung mit dem darunter liegenden metallischen Substrat eine hohe Haftfestigkeit auf (vgl. Fig. 5). Diese Oberflächenstrukturierung 4 führt auch zu einer wesentlichen Vergrößerung der realen Oberfläche mindestens um den Faktor 2. Dies ist wiederum eine wesentliche Voraussetzung für eine hohe Benetzungsfähigkeit.

Der so behandelte Marker wird 5 anschließend in eine Beschichtungslösung getaucht, die einem der oben dargestellten Beispiele entsprechen kann. Um die in der porösen Oberfläche 4 enthaltenden Luftblasen zu entfernen, erfolgt dieser Tauchprozess wiederum in einem Exsikkator der mit einem Unterdruck < 200 mbar beaufschlagt wird. Nach einer Behandlungszeit zwischen 2 und 5 min ist der Entgasungsprozess abgeschlossen und es sind keine aufsteigenden Luftblasen mehr sichtbar. Die Markeroberfläche 4 ist mit dem quellfähigen Polymer 3 benetzt und wird aus der Beschichtungslösung entnommen. Gegebenenfalls erfolgt anschließend eine thermisch initiierte Polymerisation bei >50°C, eine Lösungsmittelentfernung im Vakuumtrockenschrank oder eine direkte Verklebung im jeweiligen Eyelet 2.

Nach diesem Prozess wird der Marker 5 in dem Eyelet 2 positioniert. Ggf. wurde die Eyeletumgebung vorher mit der identischen (zuvor zum Beschichten des jeweiligen Ta-Markers 5) verwendeten Lösung zur quellfähigen Beschichtung belegt. Nach der Positionierung des jeweiligen Markers 5 im zugeordneten Eyelet 2 erfolgt die finale thermische Behandlung, die zu einer Polymerisation der Beschichtung auf der Röntgenmarkeroberfläche führt und eine ausreichende Haltekraft des Markers 5 im Eyelet 2 bewirkt. Danach erfolgt die Montage des Scaffolds 1 im Kathetersystem.

Gemäß einem weiteren Beispiel der Erfindung wurde ein Scaffold 1 mit einem Röntgenmarker 5 aus Gold (Au) bereitgestellt, der eine mittels Mikrostrahlen aufgeraute makroporöse Oberfläche 4 aufweist. Dieses Gerüst 1 besteht wiederum gemäß Fig. 4 aus einer degradierbaren Magnesiumlegierung und weist jeweils am distalen und am proximalen Ende 102 ein Doppeleyelet 2 auf, das für eine Befüllung mit Röntgenmarkern 5 vorgesehen ist. Der Durchmesser eines Eyelets 2 beträgt z. B. ca. 350 µm. In diese Eyelets 2 werden runde Massivmarker 5 aus Gold montiert. Die Dicke dieser Goldmarker ist identisch mit der Wanddicke des Scaffolds 1 und beträgt beispielsweise 100 µm. Der Durchmesser ist ca. 20 µm geringer als der Eyeletdurchmesser. Der Goldmarker 5 wurde vorher mittels Mikrostrahlen mit Korundkörnern aufgeraut. Die dabei erzielte Oberflächenrauheit beträgt zwischen Ra 0,5 µm und Ra 3,0 µm. Die Oberfläche des Markers 5 erhält dadurch eine mikroraue poröse Oberfläche 4. Diese Oberflächenstrukturierung führt auch zu einer wesentlichen Vergrößerung der realen Oberfläche mindestens um den Faktor 2. Dies ist eine wesentliche Voraussetzung für eine hohe Benetzungsfähigkeit.

Der so behandelte Marker wird anschließend in eine Lösung zur quellfähigen Beschichtung getaucht, die gemäß einem der oben genannten Beispiele ausgebildet sein kann. Um die in der porösen Oberfläche 4 enthaltenden Luftblasen zu entfernen erfolgt dieser Tauch-prozess wiederum bevorzugt in einem Exsikkator der mit Unterdruck < 200 mbar beaufschlagt wird. Nach einer Behandlungszeit zwischen 2 und 5 min ist der Entgasungsprozess abgeschlossen und es sind keine aufsteigenden Luftblasen mehr sichtbar. Die Markeroberfläche 4 ist mit dem quellfähigen Polymer 3 benetzt und wird aus der Beschichtungslösung entnommen. Gegebenenfalls erfolgt anschließend eine thermisch initiierte Polymerisation bei > 50°C, eine Lösungsmittelentfernung im Vakuumtrockenschrank oder eine direkte Verklebung im jeweiligen Eyelet 2.

Nach diesem Prozess wird der jeweilige Marker im entsprechenden Eyelet 2 positioniert. Ggf. wurde die Eyeletumgebung vorher mit der identischen Lösung zur quellfähigen Beschichtung, die zuvor zum Beschichten der Marker 5 verwendet wurde, belegt. Nach der Positionierung des jeweiligen Markers 5 im zugeordneten Eyelet 2 erfolgt die finale thermische Behandlung, die zu einer Polymerisation des Werkstoffverbundes führt und eine ausreichende Haltekraft der Marker 5 in den Eyelets 2 bewirkt. Danach erfolgt wiederum die Montage des Scaffolds 1 im Kathetersystem.

Gemäß einem weiteren Beispiel der Erfindung wurde ein Scaffold 1 mit einem Röntgenmarker 5 aus Platin (Pt) bereitgestellt, der eine mittels Mikrostrahlen aufgeraute makroporöse Oberfläche 4 aufweist. Dieses Gerüst 1 besteht wiederum aus einer degradierbaren Magnesiumlegierung (vgl. Fig. 4) und weist jeweils am distalen und am proximalen Ende 102 ein Doppeleyelet 2 auf, das für eine Befüllung mit Röntgenmarkern 5 vorgesehen ist. Der Durchmesser eines Eyelets 2 beträgt z. B. ca. 350 µm. In diese Eyelets 2 werden runde Massivmarker 5 aus Platin montiert. Die Dicke dieser Platinmarker 5 ist identisch mit der Wanddicke des Scaffolds 1 und beträgt beispielsweise 100 µm. Der Durchmesser ist ca. 20 µm geringer als der Eyeletdurchmesser. Der jeweilige Platinmarker 5 wurde vorher mittels Mikrostrahlen mit Korundkörnern aufgerauht. Die dabei erzielte Oberflächenrauheit beträgt zwischen Ra 0,5 µm und Ra 3,0 µm. Die Oberfläche 4 des jeweiligen Markers 5 erhält dadurch eine mikrorauhe poröse Oberfläche 4. Diese Oberflächenstrukturierung führt auch zu einer wesentlichen Vergrößerung der realen Oberfläche mindestens um den Faktor 2. Dies ist eine wesentliche Voraussetzung für eine hohe Benetzungsfähigkeit.

Der so behandelte jeweilige Marker 5 wird anschließend in eine Lösung zur quellfähigen Beschichtung getaucht, die gemäß einem der obigen Beispiele ausgeführt ist. Um die in der porösen Oberfläche 4 enthaltenden Luftblasen zu entfernen, erfolgt dieser Tauchprozess in einem Exsikkator der mit Unterdruck < 200 mbar beaufschlagt wird. Nach einer Behandlungszeit zwischen 2 und 5 min ist der Entgasungsprozess abgeschlossen und es sind keine aufsteigenden Luftblasen mehr sichtbar. Die Markeroberfläche 5 ist mit dem quellfähigen Polymer 3 benetzt und wird aus der Beschichtungslösung entnommen. Gegebenenfalls erfolgt anschließend eine thermisch initiierte Polymerisation bei > 50°C, eine Lösungsmittelentfernung im Vakuumtrockenschrank oder eine direkte Verklebung im jeweiligen Eyelet 2.

Nach diesem Prozess wird der jeweilige Marker im betreffenden Eyelet 2 positioniert. Ggf. wurde die Eyeletumgebung vorher mit der zuvor zum Beschichten der Marker 5 verwendeten identischen Lösung zur quellfähigen Beschichtung belegt. Nach der Positionierung des jeweiligen Markers 5 im zugeordneten Eyelet 2 erfolgt die finale thermische Behandlung, die zu einer Polymerisation der Beschichtung auf der Röntgenmarkeroberfläche führt und eine ausreichende Haltekraft des jeweiligen Markers im zugeordneten Eyelet 2 bewirkt. Danach erfolgt wiederum die Montage des Scaffolds 1 im Kathetersystem.

Gemäß einem weiteren Beispiel wird ein Scaffold 1 mit einem Röntgenmarker 5 aus einem röntgenabsorbierenden Metall wie z. B. Pt, Au, W, Ta, oder Ir vorgeschlagen, der eine dünne silikatische und damit nichtleitende Oberfläche 4 aufweist.
Diese silikatische Oberfläche 4 wird durch Eintauchen von Drähten aus Röntgenmarkermaterialien in verdünnte Wasserglaslösungen und anschließende Lufttrocknung erzielt. Dabei bilden sich in Abhängigkeit des Verdünnungsgrades des Wasserglases ca. 1 µm bis 10 µm dicke und elektrisch isolierende Schichten auf den Drahtoberflächen. Die Zusammensetzung dieser Schichten entspricht der Formel M₂O ^{∗} nSiO₂. Dabei kann M aus den Elementen Li, Na oder K bestehen. Die Verwendung von Li-haltigen Wasserglaslösungen bietet den Vorteil, dass die entstehenden Schichten nach dem Trocknungsprozess eine geringere Neigung zur Rissbildung aufweisen. Die so behandelten Drähte werden danach (z. B. mit dem Microtom oder mit einer mit mikrokristallinen Diamantkörnern besetzten Drahtsäge) in ca. 100 µm dünne Abschnitte getrennt und wie in einem der oben genannten Beispiele weiter behandelt um zu einem Gerüst 1 mit einem beschichteten Röntgenmarker 5 zu gelangen.

Die Technologie des Trennens mit dem Microtom oder der Diamantdrahtsäge stellt sicher, dass saubere und verwerfungsfreie Schnittkanten entstehen und die Silikatschicht nicht splittert. Diese Herangehensweise allein stellt sowohl eine nicht erfindungsgemäße Alternative zum Einsatz von quellfähigen Polymeren dar und kann andererseits aber auch in Kombination mit diesen zum Einsatz gebracht werden. Das bedeutet, dass die silikathaltigen Oberflächen 4 der Röntgenmarker 5 selbst dann keine korrosionsfördernde Lokalelementwirkung entfalten, wenn der Klebespalt keinen oder nicht genügend Klebstoff enthält.

Im Ergebnis ermöglicht die Erfindung mit Vorteil eine Vermeidung der Gefahr einer unbeabsichtigt und plötzlich auftretenden Bildung von Lokalelementen während der klinisch relevanten Stützzeit des Gerüsts und die daraus resultierende nicht kalkulierbare Korrosion mit verstärkter lokaler Wasserstoffbildung und damit verbundener unerwünschter biologischer Reaktionen im Körper des Patienten.

## Patentansprüche

1. Medizinisches Implantat, mit:
- einem Gerüst (1), das zumindest eine Aufnahme (2) für einen Röntgenmarker (5) aufweist, wobei das Gerüst (1) ein erstes Metall aufweist,
- zumindest einem in der Aufnahme (10) angeordneten monolithischen Röntgenmarker (5), wobei der Röntgenmarker (5) ein zweites Metall aufweist,
und wobei auf den Röntgenmarker (5) zum Verhindern eines korrosionsfördernden Kontakts zwischen dem Röntgenmarker (5) und dem Gerüst (1) eine elektrisch isolierende Beschichtung (3) aufgebracht ist
**dadurch gekennzeichnet, dass** die Beschichtung (3) derart quellfähig ausgebildet ist, dass sie bei einem Kontakt mit einem Wasser enthaltenden Medium eine Volumenzunahme erfährt.

2. Medizinisches Implantat nach Anspruch 1, **dadurch gekennzeichnet, dass** der Röntgenmarker (1) eine Oberfläche (4) mit einer Porenstruktur aufweist, wobei der Porendurchmesser bevorzugt im Bereich von 0,5 Mikrometer bis 3 Mikrometer liegt, oder dass der Röntgenmarker (1) eine Oberfläche (4) mit einer Oberflächenstruktur aufweist, die bevorzugt einen Oberflächenrauheitswert Ra im Bereich von 0,5 Mikrometer bis 3 Mikrometer aufweist.

3. Medizinisches Implantat nach Anspruch 2, **dadurch gekennzeichnet, dass** die Beschichtung (3) in den Poren oder in der Oberflächenstruktur aufgenommen ist und dazu ausgebildet ist, bei einem Kontakt mit einem Wasser enthaltenden Medium aus den Poren oder der Oberflächenstruktur herauszutreten, so dass bevorzugt eine Zentrierung des Röntgenmarkers (5) in der Aufnahme (2) erfolgt.

4. Medizinisches Implantat nach einem der vorhergehenden Ansprüche, **dadurch gekennzeichnet, dass** der Röntgenmarker (5) in der Aufnahme (2) festgeklebt ist.

5. Medizinisches Implantat nach einem der vorhergehenden Ansprüche, **dadurch gekennzeichnet, dass** das zweite Metall eines der folgenden Metalle ist: Wolfram, Tantal, Gold, Platin, Iridium.

6. Medizinisches Implantat nach einem der vorhergehenden Ansprüche, **dadurch gekennzeichnet, dass** das Gerüst (1) ein degradierbares Gerüst ist.

7. Medizinisches Implantat nach einem der vorhergehenden Ansprüche, **dadurch gekennzeichnet, dass** das erste Metall Magnesium ist, wobei das Magnesium Bestandteil einer Magnesiumlegierung ist, die das Gerüst (1) aufweist oder aus der das Gerüst (1) besteht.

8. Medizinisches Implantat nach einem der vorhergehenden Ansprüche, **dadurch gekennzeichnet, dass** die Beschichtung einen oder mehrere der folgenden Stoffe aufweist: ein Polymer, insbesondere ein möglicherweise quellfähiges, unlösliches, vernetztes Polymer wie u.a. Polyacrylate, Polyacrylsäure (PAA), Polylaurinsäurevinylester, Polyvinylimidazol, Polyvinylpyrrolidon, Polyglycolsäure (PGA), Polyethylenglykol (PEG), Hyaluronsäure (HA), Polyharnstoffe, Polyurethane, Wasserglas, Cyanacrylat, Paraffin in Kombination mit einem geeigneten Vernetzer und ggf. einem geeigneten Lösungsmittel.

9. Verfahren zur Herstellung eines medizinischen Implantats, wobei ein Gerüst (1) bereitgestellt wird, das zumindest eine Aufnahme (2) für einen monolithischen Röntgenmarker (5) aufweist, wobei das Gerüst (1) ein erstes Metall aufweist, und wobei ein zweites Metall aufweisender monolithischer Röntgenmarker (5) bereitgestellt wird, auf den eine elektrisch isolierende Beschichtung (3) mittels Kontaktierung mit einer Beschichtungslösung aufgebracht wird, wobei die Beschichtungslösung ein quellfähiges Polymer aufweist und wobei der beschichtete Röntgenmarker in der Aufnahme festgeklebt wird.

10. Verfahren nach Anspruch 9, **dadurch gekennzeichnet, dass** der Röntgenmarker (5) durch eine Behandlung der Oberfläche (4) des Röntgenmarkers (5) eine Poren- oder Oberflächenstruktur erhält.

11. Verfahren nach Anspruch 9 oder 10, **dadurch gekennzeichnet, dass** die Beschichtungslösung ein Hydrogelmonomer, einen Vernetzer, einen Polymerisationsinitiator und einen Polymerisationskatalysator aufweist.

12. Verfahren nach einem der Ansprüche 9 bis 11, **dadurch gekennzeichnet, dass** der Röntgenmarker (5) in der Aufnahme (2) mittels eines Klebstoffs festgeklebt wird, wobei der Klebstoff insbesondere durch die Beschichtungslösung gebildet wird.

## Claims

1. A medical implant comprising:
- a framework (1) which comprises at least one receptacle (2) for an X-ray marker (5), the framework (1) comprising a first metal, and
- at least one monolithic X-ray marker (5) arranged in the receptacle (10), the X-ray marker (5) comprising a second metal,
wherein an electrically insulating coating (3) is applied to the X-ray marker (5) in order to prevent corrosion-promoting contact between the X-ray marker (5) and the framework (1),
**characterised in that** the coating (3) is swellable, in such a way that it experiences an increase in volume in the event that it comes into contact with a water-containing medium.

2. The medical implant according to claim 1, **characterised in that** the X-ray marker (1) has a surface (4) with a pore structure, the pore diameter preferably lying in the range of from 0.5 micrometres to 3 micrometres, or **in that** the X-ray marker (1) has a surface (4) with a surface structure which preferably has a surface roughness value Ra in the range of from 0.5 micrometres to 3 micrometres.

3. The medical implant according to claim 2, **characterised in that** the coating (3) is received in the pores or in the surface structure and is designed, in the event of contact with a water-containing medium, to egress from the pores or the surface structure so that the X-ray marker (5) is preferably centred in the receptacle (2).

4. The medical implant according to any one of the preceding claims, **characterised in that** the X-ray marker (5) is glued in the receptacle (2).

5. The medical implant according to any one of the preceding claims, **characterised in that** the second metal is one of the following metals: tungsten, tantalum, gold, platinum, iridium.

6. The medical implant according to any one of the preceding claims, **characterised in that** the framework (1) is a degradable framework.

7. The medical implant according to any one of the preceding claims, **characterised in that** the first metal is magnesium, the magnesium being part of a magnesium alloy which the framework (1) comprises or which the framework (1) consists of.

8. The medical implant according to any one of the preceding claims, **characterised in that** the coating comprises one or more of the following materials: a polymer, in particular a possibly swellable, insoluble, cross-linked polymer, such as, *inter alia,* polyacrylates, polyacrylic acid (PAA), polylauric acid vinyl ester, polyvinylimidazole, polyvinylpyrrolidone, polyglycolic acid (PGA), polyethylene glycol (PEG), hyaluronic acid (HA), polyureas, polyurethanes, waterglass, cyanoacrylate, and paraffin in combination with a suitable cross-linker and possibly a suitable solvent.

9. A method for producing a medical implant, wherein a framework (1) is provided which comprises at least one receptacle (2) for a monolithic X-ray marker (5), the framework (1) comprising a first metal, and a monolithic X-ray marker (5) comprising a second metal being provided, with an electrically insulating coating (3) being applied to said X-ray marker by contact with a coating solution, the coating solution comprising a swellable polymer, and the coated X-ray marker being glued in the receptacle.

10. The method according to claim 9, **characterised in that** the X-ray marker (5) is provided with a pore structure or surface structure as the result of a treatment of the surface (4) of the X-ray marker (5).

11. The method according to claim 9 or 10, **characterised in that** the coating solution comprises a hydrogel monomer, a cross-linker, a polymerisation initiator and a polymerisation catalyst.

12. The method according to any one of claims 9 to 11, **characterised in that** the X-ray marker (5) is glued in the receptacle (2) by means of an adhesive, the adhesive being formed in particular by the coating solution.

## Revendications

1. Implant médical avec:
- une ossature (1) qui présente au moins un réceptacle (2) pour un marqueur de rayons X (5), où l'ossature (1) présente un premier métal,
- au moins un marqueur de rayons X (5) monolithique disposé dans le réceptacle (10), où le marqueur de rayons X (5) présente un deuxième métal,
et dans lequel un revêtement (3) isolant électriquement est rapporté sur le marqueur de rayons X (5) pour empêcher un contact favorisant la corrosion entre le marqueur de rayons X (5) et l'ossature (1)
**caractérisé en ce que** le revêtement (3) est susceptible de se gonfler de telle manière qu'il subit une augmentation de volume lors d'un contact avec un milieu contenant de l'eau.

2. Implant médical selon la revendication 1, **caractérisé en ce que** le marqueur de rayons X (1) présente une surface (4) avec une structure poreuse, le diamètre des pores se situant de préférence dans la plage de 0,5 micromètre à 3 micromètres, ou que le marqueur de rayons X (1) présente une surface (4) avec une structure de surface qui présente de préférence une valeur de rugosité de surface Ra dans la plage de 0,5 micromètre à 3 micromètres.

3. Implant médical selon la revendication 2, **caractérisé en ce que** le revêtement (3) est recueilli dans les pores ou dans la structure de surface et est conçu pour sortir des pores ou de la structure de surface lors d'un contact avec un milieu contenant de l'eau de telle sorte qu'il se produit dans le réceptacle (2) de préférence un centrage du marqueur de rayons X (5).

4. Implant médical selon l'une des revendications précédentes, **caractérisé en ce que** le marqueur de rayons X (5) est solidement collé dans le réceptacle (2).

5. Implant médical selon l'une des revendications précédentes, **caractérisé en ce que** le deuxième métal est l'un des métaux suivants : tungstène, tantale, or, platine, iridium.

6. Implant médical selon l'une des revendications précédentes, **caractérisé en ce que** l'ossature (1) est une ossature dégradable.

7. Implant médical selon l'une des revendications précédentes, **caractérisé en ce que** le premier métal est du magnésium, le magnésium étant le composant d'un alliage de magnésium, que présente l'ossature (1) ou à base duquel l'ossature (1) est constituée.

8. Implant médical selon l'une des revendications précédentes, **caractérisé en ce que** le revêtement présente une ou plusieurs des substances suivantes : un polymère, en particulier, un polymère réticulé si possible susceptible de se gonfler, non soluble, comme, entre autres, des polyacrylates, de l'acide polyacrylique (PAA), du polyester vinylique d'acide laurique, du polyvinyl imidazole, de la polyvinyl pyrrolidone, de l'acide polyglycolique (PGA), du polyéthylène glycol (PEG), de l'acide hyaluronique (HA), des polyurées, des polyuréthanes, d'un orthosilicate, d'un cyanoacrylate, de la paraffine, en combinaison avec un agent mouillant approprié et éventuellement un solvant approprié.

9. Procédé de fabrication d'un implant médical, dans lequel une ossature (1) est mise au point, qui présente au moins un réceptacle (2) pour un marqueur de rayons X (5) monolithique, où l'ossature (1) présente un premier métal et où un marqueur de rayons X (5) monolithique présentant un deuxième métal est mis au point, sur lequel un revêtement (3) isolant électriquement est rapporté au moyen d'un contact avec une solution de revêtement, où la solution de revêtement présente un polymère susceptible de se gonfler et où le marqueur de rayons X est solidement collé dans le réceptacle.

10. Procédé selon la revendication 9, **caractérisé en ce que** le marqueur de rayons X (5) acquiert une structure poreuse ou de surface par un traitement de la surface (4) du marqueur de rayons X (5).

11. Procédé selon la revendication 9 ou la revendication 10, **caractérisé en ce que** la solution de revêtement présente un hydrogel monomère, un agent mouillant, un initiateur de polymérisation et un catalyseur de polymérisation.

12. Procédé selon l'une des revendications 9 à 11, **caractérisé en ce que** le marqueur de rayons X (5) est solidement collé dans le réceptacle (2) au moyen d'un adhésif, l'adhésif étant en particulier formé par la solution de revêtement.
